# EUROPEAN PATENT APPLICATION

(11) **EP 4 070 735 A1**
(43) Date of publication of application: **12.10.2022**
(21) Application number: 22167657.0
(22) Date of filing: 11.04.2022
(51) Int. Cl.: A61B 10/00

(54) **ORAL FLUID, SALIVA AND SPUTUM COLLECTION DEVICE**

(30) Priority: 09.04.2021 US 202117226980
(71) Applicant: Orasecure LLC, Mogadore OH 44260 (US)
(72) Inventor: HINES, Mary E., Mogadore, 44260 (US); EDEN, Thomas M., III, Mogadore, 44260 (US); MCCOY, Patricia G., Mogadore, 44260 (US); CORL, William, Mogadore, 44260 (US)
(74) Representative: Forresters IP LLP

(57) **Abstract**

Disclosed herein are devices and methods for collecting oral fluids, saliva, or sputum. One exemplary device includes a collection cup with a hollow needle extending from a bottom end of the cup, where the hollow needle is covered with a compressible sleeve. A clear vacuum tube is positioned beneath the hollow needle. Oral fluids, saliva, or sputum from a donor are collected into the collection cup, and the vacuum tube is pushed onto the hollow needle and the hollow needle passes through the compressible sleeve, through a seal of the vacuum tube, and into the vacuum tube. Oral fluids, saliva, or sputum from the collection cup are drawn into the vacuum tube until the vacuum in the vacuum tube dissipates. An oral fluid specimen is, thus, provided ready for laboratory transport, point of collection screening, or diagnostic analysis while the oral fluid specimen is still in the transparent vacuum tube.

## Description

### CROSS-REFERENCE TO RELATED APPLICATIONS

This application is a continuation-in-part of International Application No. PCT/US2020/029209, titled "Oral Fluid, Saliva & Sputum Collection Device and Method" and filed on April 22, 2020, which claims priority to U.S. Non-provisional Patent Application No. 16/842,220, titled "Oral Fluid, Saliva & Sputum Collection Device and Method" and filed on April 7, 2020, which claims priority to U.S. Provisional Patent Application No. 62/928,406 titled "Filtered Neat Oral Fluid Split Specimen Collection Device" and filed on October 31, 2019, the contents of each of these patent applications being incorporated herein by reference in their entirety.

### TECHNICAL FIELD

The present disclosure relates to devices for collecting oral fluids, saliva and sputum. Throughout this disclosure such fluids are collectively referred to as "oral fluids" or "specimens." All three of these fluids generated by the human body have long been found useful for determining the health of people and in particular, for the diagnosis of disease, such as severe acute respiratory syndrome coronavirus 2 (also known as SARS-CoV-2 and, commonly referred to as COVID-19), prediction of disease progression, monitoring of therapeutic drug levels and detection of illicit drugs. In particular, the present disclosure relates to an oral fluids collection device wherein the donor can discharge oral fluids directly from the donor's mouth into a container and wherein the oral fluids can be filtered and drawn into removable vacuum tubes, ready for laboratory transport, point of collection screening, testing, or diagnostic analysis while the oral fluid specimen is still in the transparent vacuum tube.

### BACKGROUND

Saliva samples are commonly collected by intra-oral sponge absorption and by direct expectoration into a container. An example of intra-oral sponge absorption is disclosed in U.S. Patent No. 4,580,577, which describes an absorbent mass (collection paddle) that is masticated by the donor until saturated. The mass is placed in a squeezing device to expel saliva into a holding chamber, out of which a test aliquot can be removed. The problem with the currently marketed collection paddle systems is that the absorbent collection paddle is placed in a particular place in the donor's mouth, is subjected to touching by the donor, comes into contact with the interior of the donor's mouth and tongue and any particulates that may remain in the donor's mouth, and collects from one region of the mouth. In addition, the donor has to place a saturated paddle in the transport tube that contains transport buffer. The transport tube may be subject to spillage and leakage during transportation to the laboratory. Some absorbent collection paddle systems may contain a cotton swab with chemicals to help excite the donor's salivary glands. These chemicals and the cotton swabs add more volume to the saliva sample and may contaminate the sample so that it may be unknown how much of the sample is actually oral fluid. In many cases the cotton swab deteriorates or separates from a plastic portion of the paddle, both while in the donor's mouth and otherwise prior to use, and the volume from one collection to the next can vary depending on how much the cotton has deteriorated. The cotton can also trap particulates from the donor's mouth, therefore adding more volume that is not saliva and could be a contaminant. Foaming and bubbling of oral fluid specimens in this process can compromise laboratory testing. Intra-oral sponge absorption does not provide a split specimen collection nor a mixed saliva specimen. The absorbent materials can cause discomfort for the donor, perhaps even precipitating a biological reaction.

The shortcomings of the current intra-oral sponge absorption were addressed by the Department of Health and Human Services' (DHHS) in its "Mandatory Guidelines for Federal Workplace Drug Testing Programs" (published in the Federal Register at 2019-22684, issued on October 25, 2019, and went into effect on January 1, 2020, the "DHHS Regulation"), which is applicable to all federal employees. This DHHS Regulation allowed for the use of neat oral fluid collection devices. The DHHS Regulation defined the term "undiluted (neat) oral fluid" as "an oral fluid specimen to which no other solid or liquid has been added. For example, see Section 2.4: "a collection device that uses a diluted (or other compartment, process, or method that modifies the volume of the testable specimen) must collect at least one (1) mL of undiluted (neat) oral fluid."

Voiding into an open container eliminates the drawbacks of oral absorption methods but may produce a specimen having unwanted particulates from the mouth and the open container can be prone to spillage. What is needed is an open container method that avoids these problems and is able to meet the regulatory requirements for a "neat oral fluid" specimen.

### SUMMARY

The present disclosure describes a device for collecting oral fluids, saliva, or sputum. The device has a tubular housing having a top end and a bottom end. A collection cup having a top end and a bottom end is attached to the top end of the tubular housing. The bottom end of the cup has one or more hollow needles or hollow, pointed, sharp tubes extending from the bottom end of the cup into an interior of the top end of the tubular housing. The bottom end of the cup has one or more openings that open into the one or more hollow needles or hollow, pointed, sharp tubes. The hollow needles or hollow, pointed sharp tubes are covered with a penetrable sleeve to prevent unwanted drainage. One or more vacuum tubes are positioned in the housing. The vacuum tubes have a top end with a penetrable seal and a bottom end. When one or more vacuum tubes are pushed towards the top end of the housing the penetrable seals of the vacuum tubes are penetrated by the hollow needles or hollow, pointed, sharp tubes.

A base is attached to the bottom end of the housing. The base has a vacuum tube holder for the vacuum tubes. In one embodiment the base is constructed to screw into and off of the bottom end of the housing. When the base is screwed into the bottom end of the housing the vacuum tubes are moved upward towards the top of the housing, and the hollow needles or hollow, pointed, sharp tubes are pushed through the penetrable seal of the vacuum tubes. In another embodiment, the base is constructed such that the base slides upward relative to the housing. When the base is slid upward into the bottom end of the housing, the vacuum tubes are pushed upward towards the top of the housing, and the hollow needles or hollow, pointed, sharp tubes are pushed through the penetrable seal of the vacuum tubes. The device can include a removeable tab that is positioned to restrict the base from moving upward into the bottom end of the housing. This tab is kept in place during initial shipping and storage of the device and during the initial collection of a specimen. Once the specimen has been deposited into the collection cup, the tab is removed, which facilitates the movement of the base upward relative to the housing.

A filter may be positioned in an interior of the collection cup to filter the oral fluids to remove bubbles, foam, particulates and other undesirable substances. Additionally, a fill control line may be positioned on the collection cup as a guide when a donor is depositing a specimen into the device.

An advantage of the oral fluid, saliva, and sputum collection device is the collection of a specimen ready for laboratory transport, point of collection screening, testing, or diagnostic analysis while the oral fluid specimen is still in the transparent vacuum tube.

Another advantage is a collection of an oral fluid specimen in a transparent vacuum tube which allows for the use of lasers, fluorescence, and all nanotechnology diagnostic and molecular imaging of nanoparticle options currently in existence or which might exist in the future.

Another advantage is an oral fluid collection device that will reduce overall cost of oral fluid drug testing, human factor errors, opportunities for collector error, opportunities for specimen adulteration by the donor, and incidents of specimen leakage in transport.

Another advantage is a collection of an oral fluid specimen that will provide superior drug recovery characteristics for laboratory testing and/or point of collection screening in accordance with DHHS Regulations and in any application where oral fluid testing is conducted.

Another advantage is that the device allows for a self-collection by donors of their oral fluids in their homes, workplaces, physician's offices, clinics, as well as a collector directed or an observed collection, all without ever requiring the collector to come in contact with the oral fluid specimen.

Another advantage is that the device allows for the collection of an oral fluid sample from a donor, where that oral fluid sample is filtered to remove unwanted particulates and other substances and split into two neat specimens useful in testing and analysis. Such undiluted neat split specimens allow for confirmation of analyses (such as a positive or negative diagnosis or results), as a backup specimen in case of an error in testing or inconclusive result, if a donor disputes the results of the initial test, and other such circumstances.

### BRIEF DESCRIPTION OF DRAWINGS

FIG. 1 is a top, side, perspective view of an exemplary embodiment of an oral fluid collection device of the present disclosure.
FIG. 2 is an exploded perspective view of the oral fluid collection device of FIG. 1.
FIG. 3 is a side elevation view of the oral fluid collection device of FIG. 1 in an oral fluid sampling configuration.
FIG. 4 is a side elevation view of the oral fluid collection device of FIG. 1 in a vacuum tube collection configuration.
FIG. 5 is a top, side, perspective view of another embodiment of an oral fluid collection device of the present disclosure.
FIG. 6 is an exploded perspective view of the oral fluid collection device of FIG. 5.
FIG. 7 is a side elevation view of the oral fluid collection device of FIG. 5 in an oral fluid sampling configuration.
FIG. 8 is a side elevation view of the oral fluid collection device of FIG. 5 in a vacuum tube collection configuration.
FIG. 9 is a side cross-sectional view of a portion of the exemplary oral fluid collection device using a luer lock mechanism to connect a hollow needle to a collection cup.
FIG. 10 is a schematic illustration of another exemplary embodiment of an oral fluid collection device of the present disclosure.
FIG. 11 is a schematic illustration of the oral fluid collection device of FIG. 10 in preparation for use by a donor.
FIG. 12 is a schematic illustration of the oral fluid collection device of FIG. 10 in use by a donor.
FIG. 13 is a schematic illustration of the oral fluid collection device of FIG. 10 in use by a donor.
FIG. 14 is a schematic illustration of the oral fluid collection device of FIG. 10 in use by a donor.
FIG. 15 is a schematic illustration of the oral fluid collection device of FIG. 10 in use by a donor.
FIG. 16 is a schematic illustration of the oral fluid collection device of FIG. 10 in use by a donor.
FIG. 17 is a schematic illustration of the oral fluid collection device of FIG. 10 in use by a donor.
FIG. 18 is a schematic illustration of the oral fluid collection device of FIG. 10 in use by a donor.
FIG. 19 is a schematic illustration of the oral fluid collection device of FIG. 10 in use by a donor.
FIG. 20 is a schematic illustration of the oral fluid collection device of FIG. 10 in use by a donor.
FIG. 21 is a schematic illustration of the oral fluid collection device of FIG. 10 in use by a donor.
FIG. 22 is a schematic illustration of an exemplary base for use with an oral fluid collection device with a vacuum tube engaged with the base.
FIG. 23 is a schematic illustration of the exemplary base of FIG. 22, with another vacuum tube engaged with the base.
FIG. 24 is a schematic illustration of an exemplary collection cup and related accessories for use with an oral fluid collection device.
FIG. 25 is a schematic illustration of the exemplary collection cup of FIG. 24 in use with the exemplary base of FIG. 22.
FIG. 26 is a schematic illustration of the exemplary collection cup of FIG. 24 in use with the exemplary base of FIG. 22.
FIG. 27 is a schematic illustration of the exemplary collection cup of FIG. 24 in use with the exemplary base of FIG. 22.
FIG. 28 is a schematic illustration of the exemplary collection cup of FIG. 24 in use with the exemplary base of FIG. 22.
FIG. 29 is a schematic illustration of the exemplary collection cup of FIG. 24 in use with the exemplary base of FIG. 22.
FIG. 30 is a schematic illustration of the exemplary collection cup of FIG. 24 in use with the exemplary base of FIG. 22.
FIG. 31 is a schematic illustration of the exemplary collection cup of FIG. 24 in use with the exemplary base of FIG. 22.

### DETAILED DESCRIPTION OF THE DISCLOSURE

While the following description details the preferred embodiments of the present disclosure, it is to be understood that the disclosure is not limited in its application to the details of arrangement of the devices, parts, components, and methods described and illustrated in the accompanying figures, since the disclosure is capable of applying to other embodiment of oral fluid collection devices, parts, components, and methods of practice.

The present disclosure provides a rapid, safe, secure, and simultaneous split specimen collection device, for self-collection (or collector observed collection) of neat filtered saliva, oral fluid, and sputum specimens, which are transported into test tubes ready for prompt diagnostic screening and testing of a virus such as COVID-19, and of any other substances of interest in these specimens. This oral fluid collection device can provide a neat simultaneous split oral fluid specimen in full compliance with the DHHS Regulations. The due process value in regulated workplace testing (including eventually testing under U.S. Department of Transportation rules and regulations) is to allow the donor who disagrees with the result of a test to demand confirmation testing at a second Substance Abuse and Mental Health Services Administration ("SAMHSA") certified laboratory using the untested split sample. Additionally, in a non-regulated testing situation, the split specimen tube can be used for the analysis of other substances of interest, such as DNA, to prove that the specimen was actually the claimed donor's specimen where a self-collection is conducted, or similar to a situation where numerous vacuum blood tubes are simultaneously taken for diagnostic analysis.

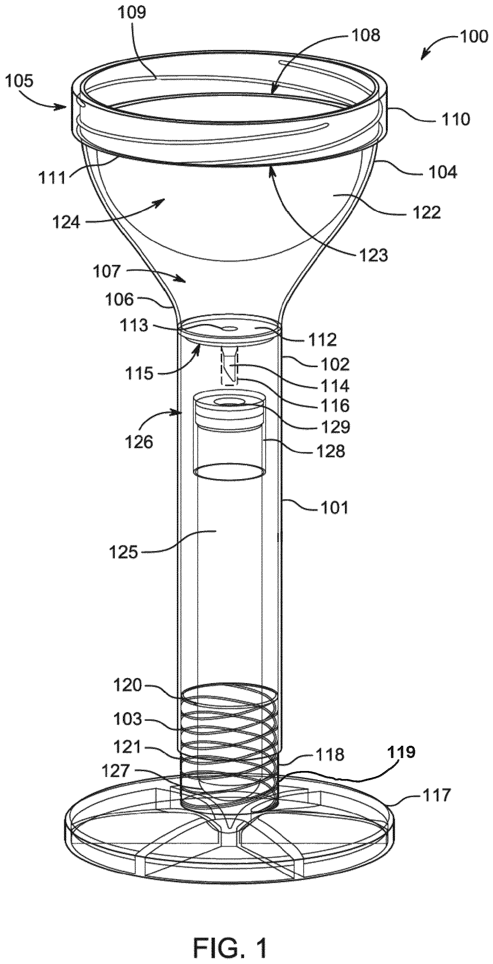

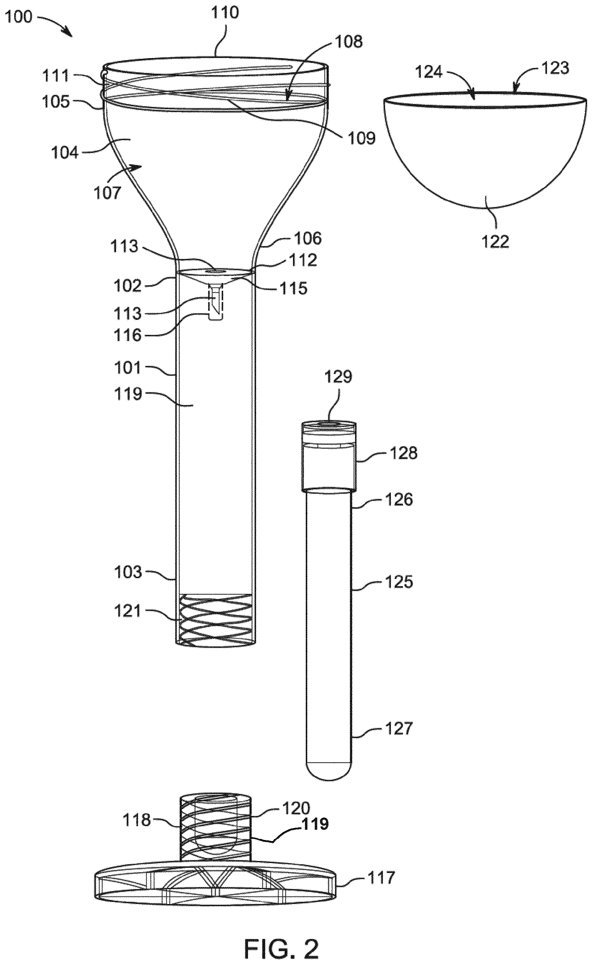

FIG. 1 is a top, side, perspective view of an exemplary oral fluid collection device **100** of the present disclosure, and FIG. 2 is an exploded perspective view of the oral fluid collection device. The collection device **100** has a hollow tubular housing **101** with a top end **102** and a bottom end **103** and a collection cup **104** with a top end **105** and a bottom end **106.** The collection cup **104** has an interior **107** and an opening **108** at the top end **105** that provides access into the interior **107** of the collection cup **104**. The top end **105** of the collection cup **104** has threads **109** around the opening **108** that are arranged to engage a screw-on cap **110** with corresponding threads **111**.

The bottom end **106** of the collection cup **104** has an opening **113** for insertion of a hollow needle **114**, to facilitate the drainage of liquid through the opening **113** and into the hollow needle **114**. The hollow needle **114** extends downward beyond the underside **115** of the bottom end **106** of the collection cup **104** and is covered with a flexible, compressible, penetrable sleeve **116** that prevents unwanted drainage through the hollow needle **114**. The hollow needle **114** can take on other arrangements, such as a hollow, pointed, sharp tube; a shard of hollow metal or plastic; or any arrangement that can facilitate the flow of fluids from one location of the other. However for convenience, such components will be referred to as hollow needles throughout this disclosure.

The collection device **100** includes a base **117**, where the base **117** includes a stem **119**. The base **117** further includes a vacuum tube holder **118** formed by the interior surface of the stem **119** and located generally in the center of the base **117**. The bottom end **103** of the housing **101** includes a threaded section **121** along the interior of the housing **101**. The exterior of the stem **119** includes corresponding threads **120** for engaging with the threads **121** at the bottom end **103** of the housing **101**. The threads **120** of the base **117** can be engaged with the threads **121** of the base **117**, and as the threads (**120** and **121**) engaged and the base **117** is screwed into the housing **101**, the vacuum tube holder **118** advances upwards towards the top end **102** of the housing **101**. As will be further described, when a vacuum tube **125** is positioned within the vacuum tube holder **118**, the vacuum tube **125** will advance upward toward the top end **102** of the housing **101** and toward the hollow needle **114**.

The oral fluid collection device **100** has a sieve or filter **122** which has an open end **123** that opens into an interior **124** of the filter **122**. The filter **122** is placed inside the interior **107** of the collection cup **104** and, preferably, has a shape which conforms to the shape of the collection cup **104**. The open end **123** of the filter **122** is adjacent to the opening **108** of the collection cup **104** at the top end **105** of the collection cup **104**. The filter **122** is made of a non-absorbent material such as plastic or metal but paper can also be used. Preferably, the filer is a microfilter. The microfilter is a micro-filtration screen made from a specific weave type, wire diameter, and mesh count for the filtration of suspended solid particles, bubbles and viscous matter in order to provide the ideal fluid specimen. For example, a type 304 stainless steel mesh is used having 0.0098 inch openings with a 0.0037 inch wire diameter. The microfilter is precisely cut and formed to fit the collection cup **104**.

A vacuum tube **125** is positioned in the interior of the housing **101**. The vacuum tube **125** has a top end **126** and a bottom end **127**. The top end **126** is open and has a cap **128** which covers the top end **126**. The cap **128** has a flexible penetrable membrane **129** which can be punctured by the hollow needle **114**. The compressible sleeve **116** on the hollow needle **114** can also be punctured by the hollow needle **114**. Vacuum tubes for collection fluid samples are well known in the art and can be obtained commercially. Flexible, compressive sleeves for the hollow needle **114** can also be obtained commercially.

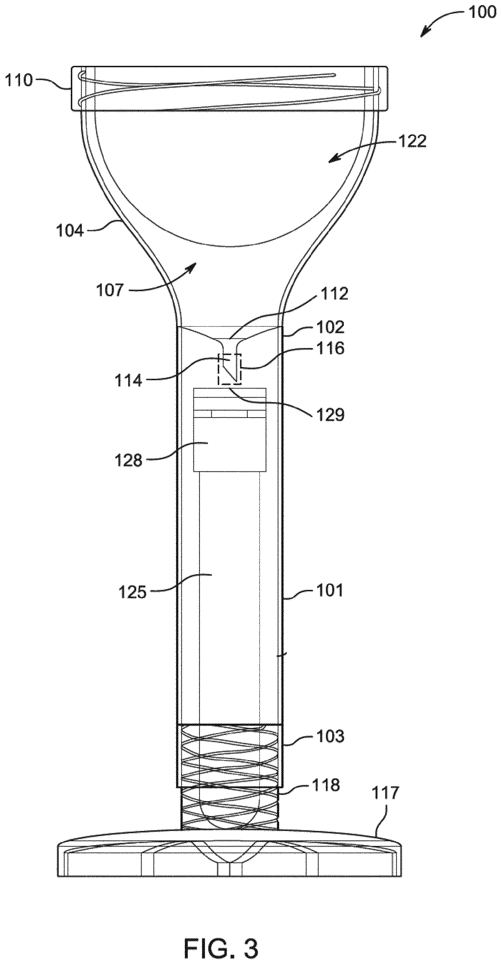

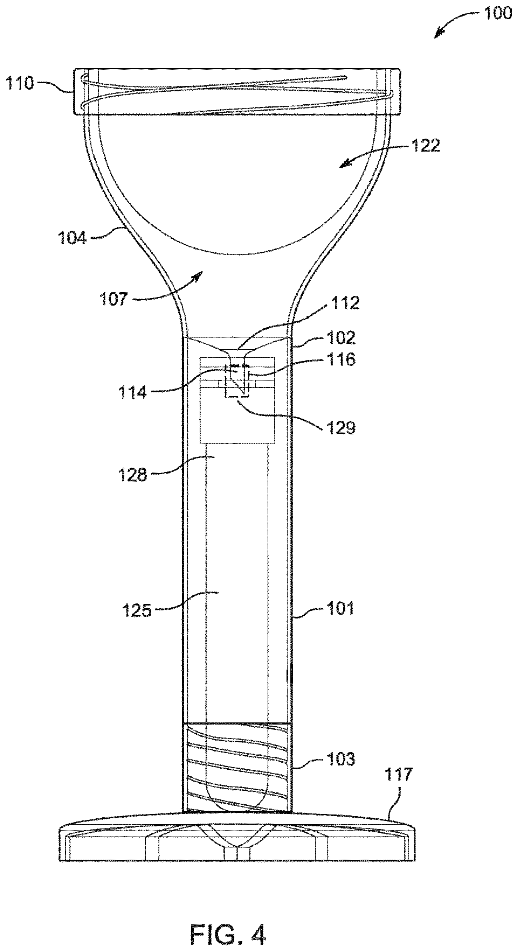

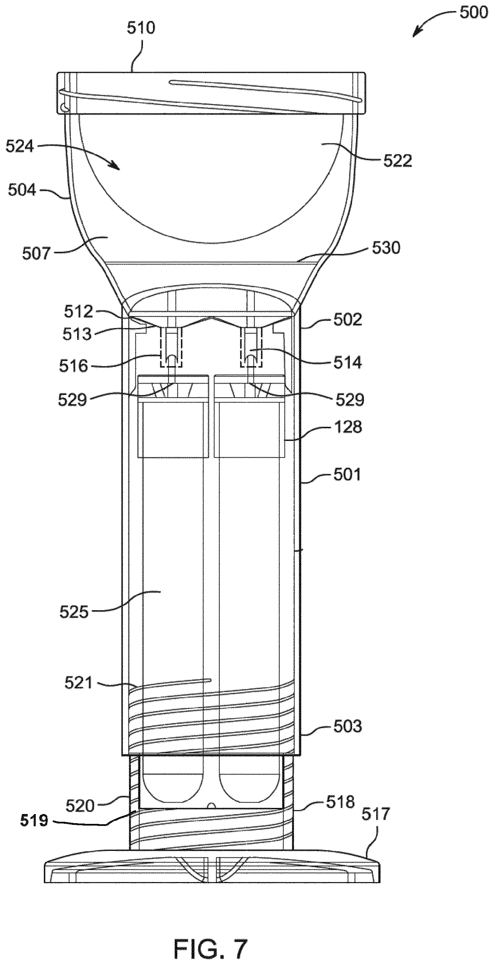

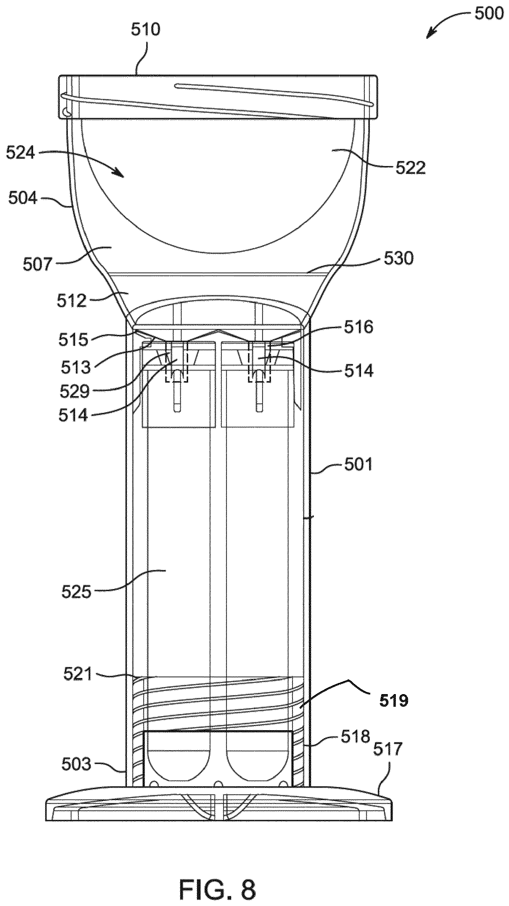

FIG. 3 is side elevation view of the oral fluid collection device in an oral fluid sampling configuration. FIG. 4 is side elevation view of the oral fluid collection device in a vacuum tube collection configuration. In use, the cap **110** is unscrewed from the top of the collection cup **104** and a donor voids his or her oral fluids into the filter **122** that is in the collection cup **104**. The oral fluids will drain through the anti-foaming filter **122** to the bottom of the collection cup **104** and collect there. The collection cup **104** can have a control fill line (for example, as illustrated in FIGS. 7 and 8 as reference number **530**) to guide the donor to provide a sufficient and precise amount of oral fluid. The oral fluid will also drain into the hollow needle **114** but will not flow out of the hollow needle **114** because of the blocking action of the sleeve **116** that is placed over and attached to the hollow needle **114**. Sputum can also be collected in this manner but it may be necessary for the donor or the collector to first remove the filter, which can be done manually.

After the oral fluids sample is provided, the cap **110** is replaced on the collection cup **104** and the base **117** of the housing **101** is screwed into the housing **101**. As the base **117** is screwed inward, the vacuum tube **125** will move upward and engage the hollow needle **114**. As the base **117** is further screwed inward, the cap **128** of the vacuum tube **125** engages the sleeve **116** and the hollow needle **114**, and such upward movement results in a force being applied between the hollow needle **114** and the cap **128** of the vacuum tube **125**, resulting in the hollow needle **114** penetrating both the sleeve **116** and the membrane **129** in the cap **128** of the vacuum tube **125**. The sleeve **116** compress and moves away from the tip of the hollow needle **114**, and the vacuum in the vacuum tube **125** pulls the oral fluids into the vacuum tube **125** until the vacuum is dissipated.

Once the vacuum tube **125** is properly filled, the base **117** and the housing **101** are unscrewed, causing the vacuum tube **125** to move downward relative to the housing **101**, causing the hollow needle **114** to be removed from the vacuum tube **125**. Once the hollow needle **114** is removed, the membrane **129** of the cap **128** of the vacuum tube **125** reseals the vacuum tube **125**. The sleeve **116** uncompresses, returns to its original configuration, and again covers the tip of the hollow needle **114**. Thus providing users with protection from the sharp portion of the hollow needle **114**. The vacuum tube **125** can then be removed from the housing **101** and the vacuum tube holder **118**. In one embodiment, the vacuum tube **125** can be held in the vacuum tube holder **118** using a friction fit that is arranged to retain the vacuum tube **125** in the vacuum tube holder **118**, while allowing the user to apply a reasonable force to remove the vacuum tube **125** from the vacuum tube holder **118**. The vacuum tube **125** containing the sample of oral fluids can then be transferred to a laboratory for analysis.

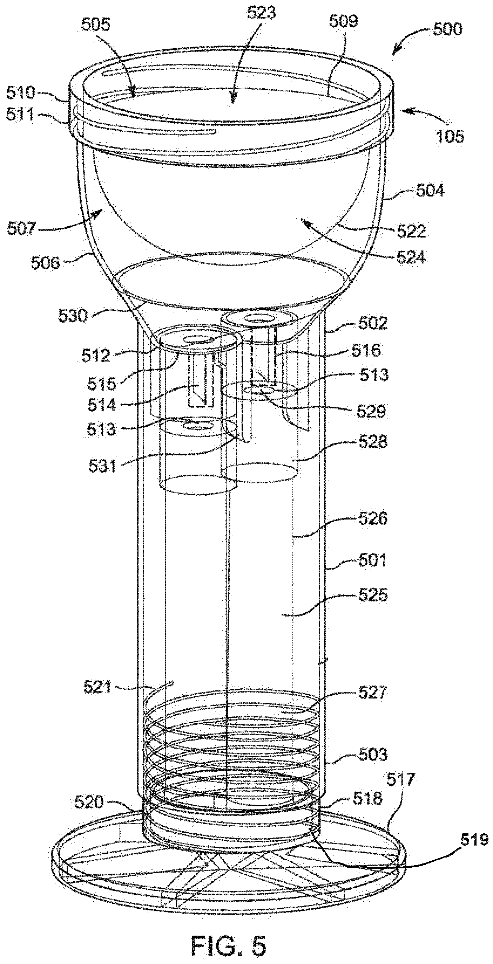

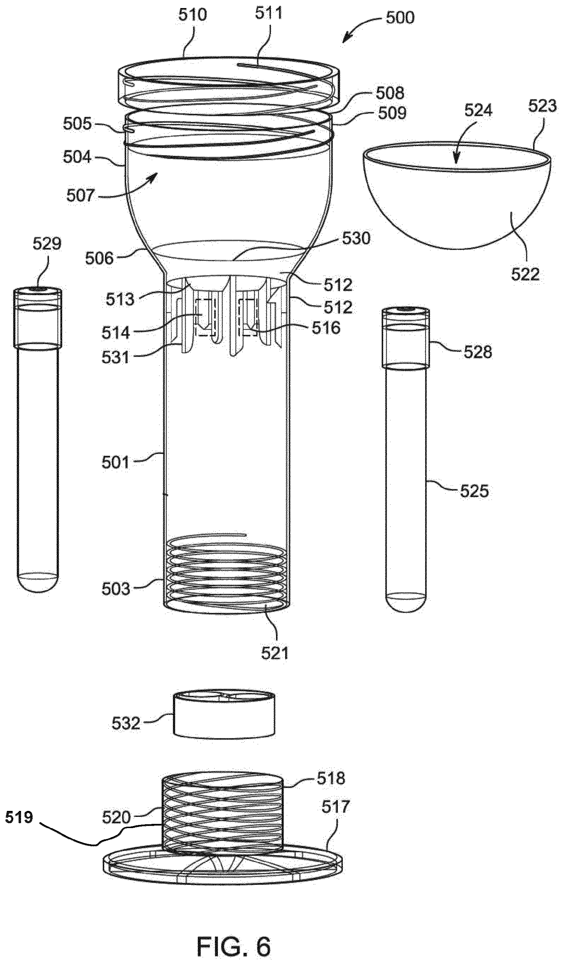

In another embodiment, the oral fluid collection device can accommodate a plurality of vacuum tubes. FIG. 5 is a top, side, perspective view of such an embodiment of an oral fluid collection device **500** with two vacuum tubes **525**. FIG. 6 is an exploded perspective view of the oral fluid collection device **500** with two vacuum tubes **525**. The oral fluid collection device **500** has a hollow tubular housing **501** with a top end **502** and a bottom end **503** and a collection cup **504** with a top end **505** and a bottom end **506**. The collection cup **504** has an interior **507** and the top end **505** forms an opening **508** into the interior **507**. The top end **505** of the collection cup **504** has threads **509** around the opening **508** engaging a screw-on cap **510** having corresponding threads **511**.

The bottom end **506** of the collection cup **504** has an pair of openings **513** for insertion of a pair of hollow needles **514** for drainage of liquid. The hollow needles **514** extend downward beyond the underside **515** of the bottom end **506** and are each covered with a flexible, compressible sleeve **516** that prevents unwanted drainage through the hollow needles **514**.

The collection device **500** includes a base **517**, where the base **517** includes a stem **519**. The base **517** further includes a vacuum tube holder **518** formed by the interior surface of the stem **519** and located generally in the center of the base **517**. The bottom end **503** of the housing **501** includes a threaded section **521** along the interior of the housing **501**. The exterior of the stem **519** includes corresponding threads **520** for engaging with the threads **521** at the bottom end **503** of the housing **501**. The threads **520** of the base **517** can be engaged with the threads **521** of the base **517**, and as the threads (**520** and **521**) are engaged and the base **517** is screwed into the housing **501**, the vacuum tube holder **518** advances upwards towards the top end **502** of the housing **501**. When a pair of vacuum tubes **525** are positioned within the vacuum tube holder **518**, the vacuum tubes **525** will advance upward toward the top end **502** of the housing **501** and toward the hollow needles **514**.

The oral fluid collection device **500** has a sieve or filter **522** which has an open end **523** that opens into an interior **524** of the filter **522**. The filter **522** is placed inside the interior **507** of the collection cup **504** and, preferably, has a shape which conforms to the shape of the collection cup **504**. The open end **523** of the filter **522** is adjacent to the opening **508** of the collection cup **504** at the top end **505** of the collection cup **504**.

Vacuum tubes **525** are positioned in the interior **519** of the housing **501**. Each vacuum tube **525** has a top end **526** and a bottom end **527**. The top end **526** is open and has a cap **528** which covers the top end **525**. The cap **528** has a flexible membrane **529**, which can be punctured by the hollow needle **514**. The compressible sleeve **516** on the hollow needle **514** can also be punctured by the hollow needles **514**. A fill control line **530** is located on the cup **504**, vacuum tube guides **531** are positioned at the bottom **512** of the collection cup **504**, and a vacuum tube tray **532** is shown for insertion into the vacuum tube holder **518**. The vacuum tube guides **531** and the vacuum tube tray **532** prevent the vacuum tubes **525** from rotating as the base **517** is screwed into and off of the bottom end **503** of the housing **501**. The vacuum tubes **525** may also have control fill lines to verify accuracy.

FIG. 7 is side elevation view of the oral fluid collection device **500** in an oral fluid sampling configuration. FIG. 8 is side elevation view of the oral fluid collection device **500** in a vacuum tube collection configuration. The oral fluid collection device **500** is used in a similar manner as described for the oral fluid collection device **100**.

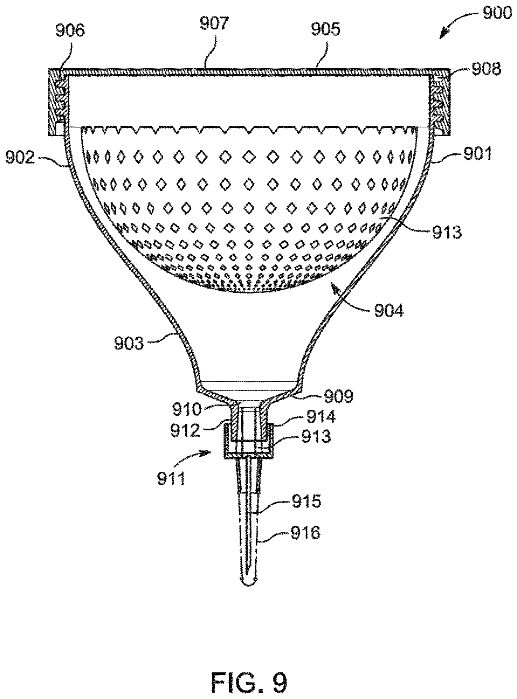

FIG. 9 is a side elevation view of an exemplary subassembly for use in an oral fluid collection device. This embodiment uses a luer lock mechanism to connect a hollow needle to a collection cup. The subassembly **900** has a collection cup **901** with a top end **902** and a bottom end **903**. The collection cup **901** has an interior **904** and the top end **902** forms an opening **905** into the interior **904**. The top end **902** of the collection cup **901** has threads **906** around the opening **905** engaging a screw-on cap **907** having threads **908**. The underside **909** of the bottom end **903** has an opening **910** and a luer lock mechanism **911** to attach a hollow needle to the opening **910**. A hollow female receptacle **912** of the luer lock mechanism **911** is attached to the opening **910**. A hollow male insert **913** of the luer lock mechanism **911** is insertable into the hollow female receptacle **912** and can be locked thereto with a locking mechanism **914**, as is well known in the art. A hollow needle **915** is attached to the hollow male insert **913** so that fluid in the collection cup **901** will flow from the collection cup **901**, through the opening **910**, through the hollow female receptacle **912**, through the hollow male insert **913**, through the hollow needle **915**, and into a vacuum tube. The subassembly **900** functions similar to the description of the oral fluid collection devices **100** and **500** for obtaining a specimen in a clear vacuum tube. In the devices **100**, **500** and subassembly **900**, the vacuum tubes can be pushed manually onto the hollow needle and can be made of any suitable plastic or metal or combination thereof.

The oral fluid collection devices **100**, **500** and subassembly **900** provide for useful methods for collecting oral fluids, saliva, or sputum. A collection cup is provided having a top end and a bottom end. The bottom end of the cup has one or more hollow needles extending from the bottom end of the cup. One or more vacuum tubes are provided. Each vacuum tube has a top end with a penetrable seal. The vacuum tubes are placed beneath the hollow needles. Oral fluids, saliva, or sputum from a donor are collected into the collection cup. The hollow needles are pushed through the penetrable seal of the vacuum tubes. The oral fluids, saliva, or sputum from the collection cup are drawn through the hollow needles and into the vacuum tubes. Preferably, the fluids, saliva, or sputum are collected up to a control line on the collection cup. If desired, a filter is placed in the collection cup and the oral fluids, saliva, or sputum are filtered before pushing the hollow needles through the penetrable seal of the vacuum tubes. A compressible sleeve can be placed over the hollow needles to prevent unwanted drainage prior to pushing the hollow needles through the penetrable seal of the vacuum tubes.

These oral fluid collection devices **100**, **500** and subassembly **900** are used to collect oral fluids, such as mixed saliva, directly from a mouth of a donor. Mixed saliva is considered a gold standard for testing saliva because it provides the purest specimen by the donor. The use of mixed saliva is in compliance with the DHHS Regulations. The oral fluid collection device **500** with a pair of vacuum tubes **525** can also provide split specimen collection.

The specimen can be initially filtered through a non-absorbent material screen at the top of the oral fluid collection cup to remove bubbles, foam, particulates, and donor induced adulterants. The collection cup can have a scratch and a sniff food grade scent sticker on its side, or supplied to the collector, to excite the donor's salivary glands so that a sufficient volume of oral fluid may be obtained to reach a control line within 5 to 10 minutes. The vacuum tubes come pre-vacuumed to draw in a precise amount of oral fluid from the cup. The precise amount is defined by laboratory requirements and/or accrediting agencies including but not limited to the DHHS Regulations and other similar regulations, such as, for example, regulations of the Federal Department of Transportation ("DOT") and similar federal and state agencies. The vacuum tubes may be prefilled with a transport and stabilizing buffer to preserve the integrity of the specimen for a number of days stated by the buffer manufacturer in an un-refrigerated state. The vacuum tubes also preserve the integrity of the specimen for transport for laboratory testing, in compliance with proposed DHHS and/or DOT split specimen collection regulations. The filtered oral fluid specimen or split specimen contained in the leak proof vacuum tubes is optimized for immediate laboratory processing or frozen storage. The vacuum tube may also be used for point of collection (POCT) screening by placement of color-coded activated reagents in the vacuum test tube. The reagents react quickly to multiple possible substances in the donor's oral fluid as the oral fluid is vacuumed from the collection cup into analytical laboratory equipment.

Advances in nanotechnology, diagnostic and molecular imaging of nanoparticles, such as by use of gold nanoparticles, quantum dots, magnetic nanoparticles, etc. combined with mobile fluorescence devices, are especially well suited to use transparent vacuum tube delivered neat oral fluid specimens. The oral fluid specimen in the transparent vacuum tube allows for the use of lasers, fluorescence, and all nanotechnology diagnostic and molecular imaging of nanoparticle options which currently exist or which might exist in the future for a true solution ("Fluorometric virus detection platform using quantum dots-gold nanocomposites optimizing the linker links variation," published in ScienceDirect Volume 1109, 1 May 2020, pages 148 - 157, found at https://doi.org/10.1016/j.aca.2020.02.039). Oral fluid has long been understood to be useful as a diagnostic tool in medicine. Technological advances over the past decades have enabled oral fluid to expand its usefulness in the diagnosis of disease, prediction of disease progression, monitoring of therapeutic drug levels and detection of illicit drugs. The easy non-invasive nature of collection and the relationship between oral fluid and plasma levels make oral fluid a valuable clinical tool. ("Oral fluid as a diagnostic tool," published in Clin Chem Lab Med 2004;42(11): 1273-1287, found at. DOI 10.1515/CCLM.2004.248).

For purposes of COVID-19 or other virus testing, the oral fluid collection devices **100, 500** and subassembly **900**, for example, allow for the safe and secure self-collection and secure transport of either saliva, oral fluids and/or sputum collected from a donor. Recently, sputum has been shown in scientific studies to have a higher recovery rate of the COVID-19 markers compared to a nasal swab, which has a higher than expected false negative rate and which is extremely invasive. ("Detection of SARS-CoV-2 in Different Types of Clinical Specimens," JAMA Research Letter published March 11, 2020 found at:25 doi:10.1001/jama.2020.3.3786 and "SARS-CoV-2 more readily detected in induced sputum than in throat swabs of convalescent COVID-19 patients," March 12, 2020 in The Lancet Infectious Diseases found at DOI: http://doi.org/10.1016/S1473-3099(20)30174-2). Likewise, there is a recent medical study showing that a saliva specimen is a consistent detection option for COVID-19 patients. The 2019 novel coronavirus (2019 - nCoV) was detected in the self-collected saliva of 91.7% (11/12) of patients. Serial saliva viral load monitoring generally showed a declining trend. Live virus was detected in saliva by viral culture. Saliva is a promising noninvasive specimen for diagnostic, monitoring, and 35 infectious control in patients with 2019-nCoV. ("Consistent Detection of 2019 Novel Coronavirus in Saliva," published 30 February 12, 2020 in the Journal for Infectious Diseases Society of America found at DOI: 10.1093/cid/ciaal49).

Because the oral fluid collection devices **100**, **500** and subassembly **900** are self-collection devices, collection of specimens can be conducted without placing a healthcare worker at risk of contracting COVID-19. The devices allow for multiple oral fluid collection possibilities based upon compliance with various pre-collection protocols and instructions from the collector. Multiple oral fluid collections make the devices ideal for collection of oral fluid specimens to measure the donor's antibody production following a bout with COVID-19. The devices are also useful following vaccination (once discovered) to determine the donor's level of immunity protection against a subsequent outbreak and to provide clearance to go safely back to work and play in a non-social distancing society.

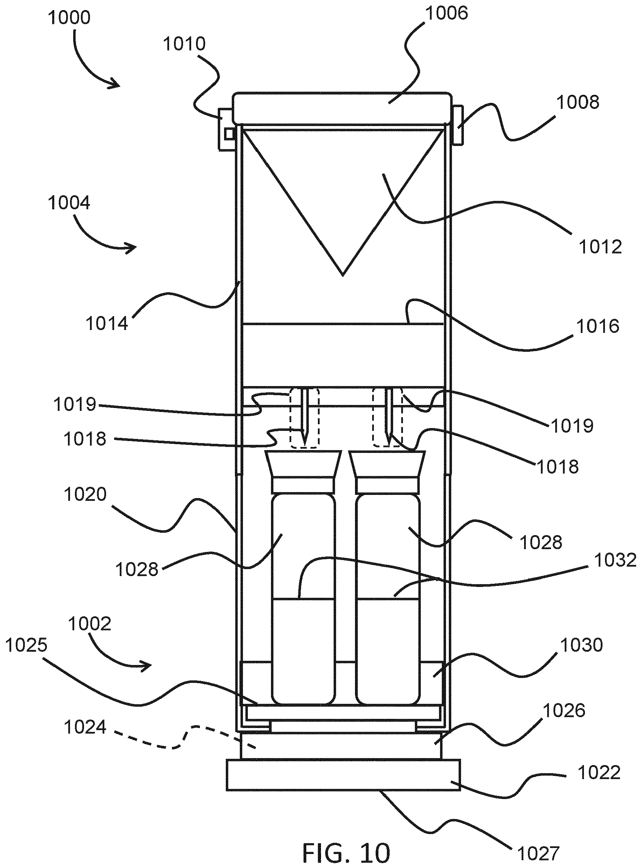

FIGS. 10-21 schematically illustrate another embodiment of an oral fluid collection device **1000**. The fluid collection device **1000** operates generally on the same principles as described herein for other oral fluid collection devices **100** and **500** and includes a pair of vacuum tubes that collect split samples. However, the oral fluid collection device **1000** illustrated in FIGS. 10-21 includes a sliding or plunger mechanism for facilitating relative linear movement between the vacuum tubes and hollow needles to collect samples in the vacuum tubes. As illustrated in FIG. 10, the oral fluid collection device **1000** includes a base **1002**, a collection cup **1004**, and a cap **1006**. The cap **1006** is connected to the collection cup **1004** by a hinge **1008** and a user can selectively open or close the cap **1006** to gain access to or secure the collection cup **1004**. Such cap **1006** is secured to the collection cup **1004** by a reversible protrusion and snap mechanism **1010**. Positioned in the collection cup **1004** is an anti-foaming filter funnel **1012** for filtering oral fluids deposited in the collection cup **1004** by a donor. A collection cup **1004** includes a thin walled body **1014** that defines the inner volume or the collection cup **1004**. A collection cup fill line **1016** can be scribed on the collection cup body **1014** to assist a user in determining when a sufficient amount of oral fluid has been deposited in the collection cup **1004**. The collection cup **1004** further includes a pair of hollow needles **1018** that are secured in a vertical position at least partially within the collection cup **1004** and in fluid communication with oral fluid deposited into the collection cup **1004**. As previously described, a flexible, compressible, penetrable sleeve **1019** can be positioned over each hollow needle **1018** to prevent unwanted drainage through the hollow needle **1018** and to offer protection to a user or donor handling the oral fluid collection device **1000** from injury from the sharp end of the hollow needle **1018**.

The base **1002** includes a thin walled body **1020** that defines the inner volume of the base **1006**. The thin walled body **1014** of the collection cup **1004** and the thin walled body **1020** of the base **1002** are reversibly secured together by a removeable seal. During the collection of oral fluids, the thin walled body **1014** of the collection cup **1004** and the thin walled body **1020** of the base **1002** remain secured together. After the collection of oral fluids is complete and the oral fluids are fully moved to and stored in vacuum tubes, the removeable seal can be removed, the collection cup **1004** and the base **1002** separated, and the vacuum tubes accessed and removed by the donor or other user of the oral fluid collection device **1000.**

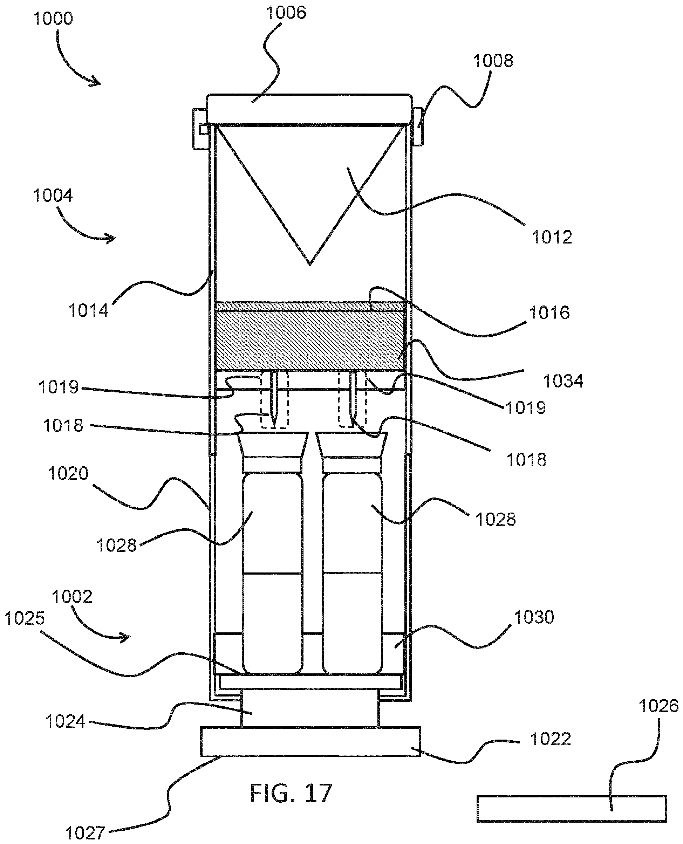

The base **1002** includes a pedestal **1022**, which forms the bottom portion of the base **1002**. The pedestal **1022** includes an upper surface **1025** and a lower surface **1027** and a recessed section **1024** located between the upper surface **1025** and lower surface **1027**_(as best illustrated in FIG. 17). As illustrated in FIG. 10, a removable tab **1026** is positioned within the recessed section **1024** and, as will be subsequently described, the removeable tab **1026** engages with the recessed section **1024** in a manner that maintains a static positional relationship between the vacuum tubes **1028** and the hollow needles **1018**. Such a static positional relationship prevents the vacuum tubes **1028** from interaction with the hollow needles **1018** prematurely (i.e., prevents accidental piercing of the membrane sealing the vacuum tubes prior to the oral fluid being in proper position to fill the vacuum tubes). As illustrated in the figures, positioned within the base **1002** are a pair of vacuum tubes **1028**, which can be optionally reversibly secured in place with a vacuum tube holder **1030**. Each vacuum tube **1028** can also include a vacuum tube fill line **1032** to inform a user whether a sufficient amount of oral fluid has been deposited into the vacuum tubes **1028**.

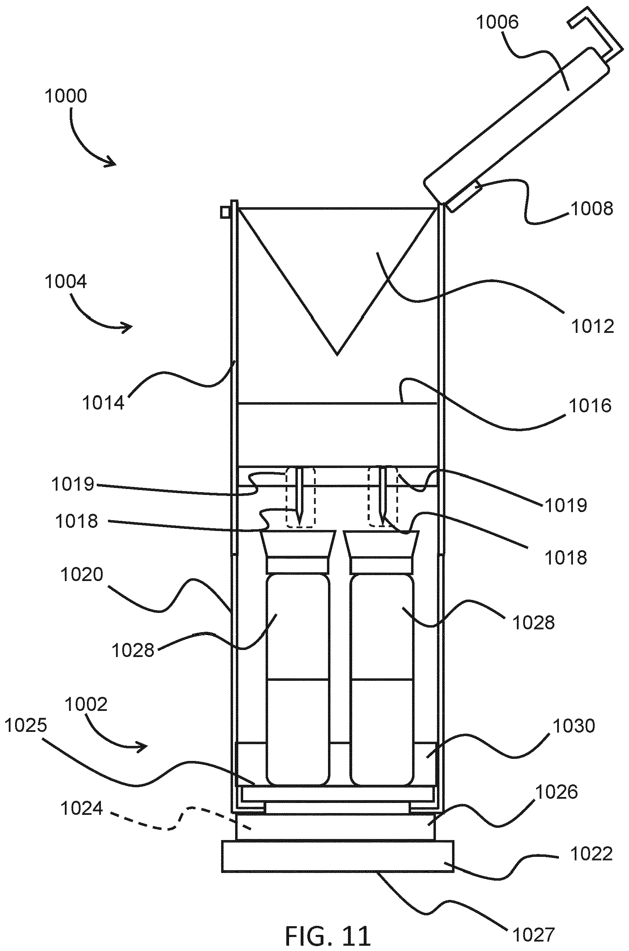

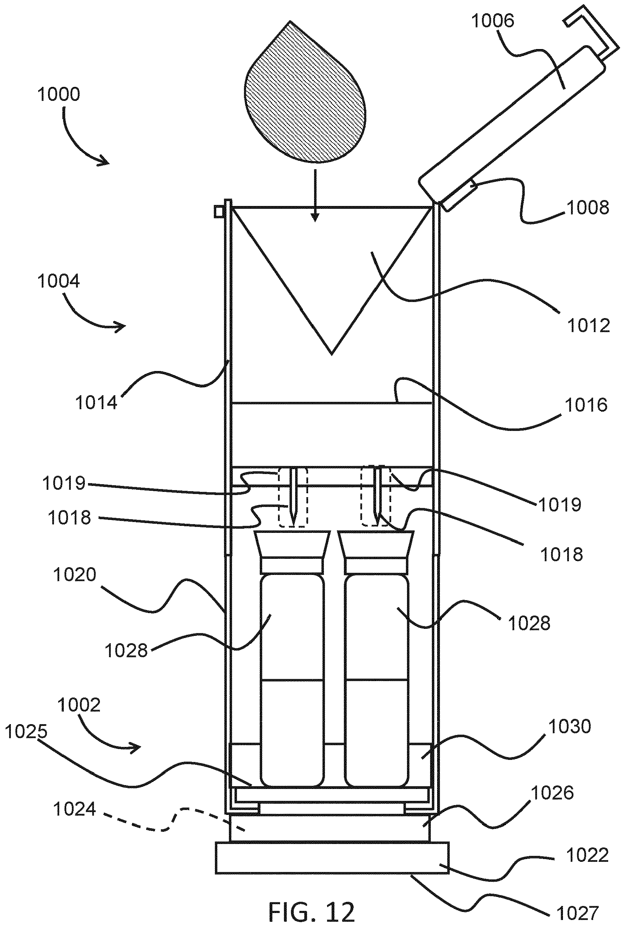

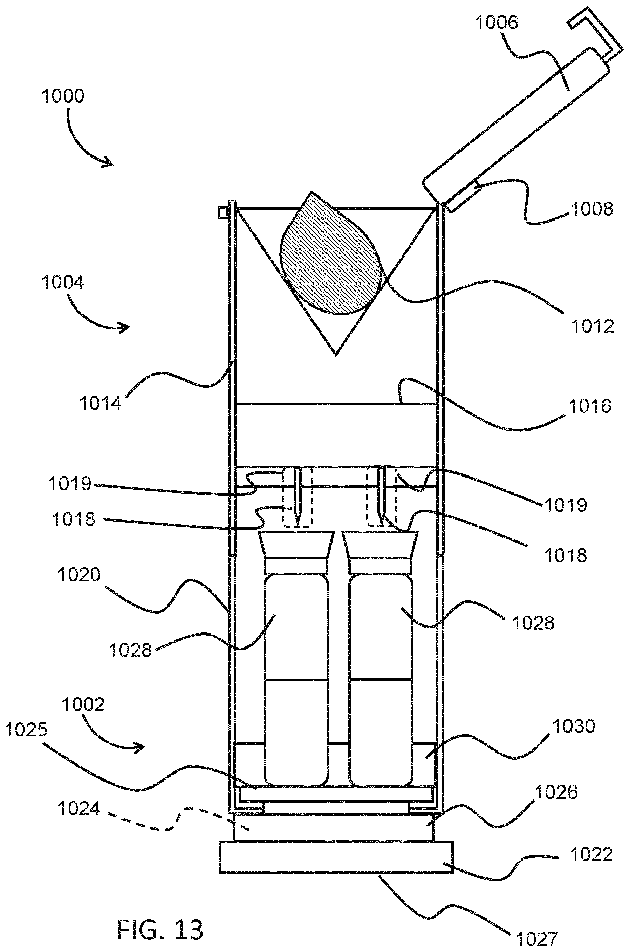

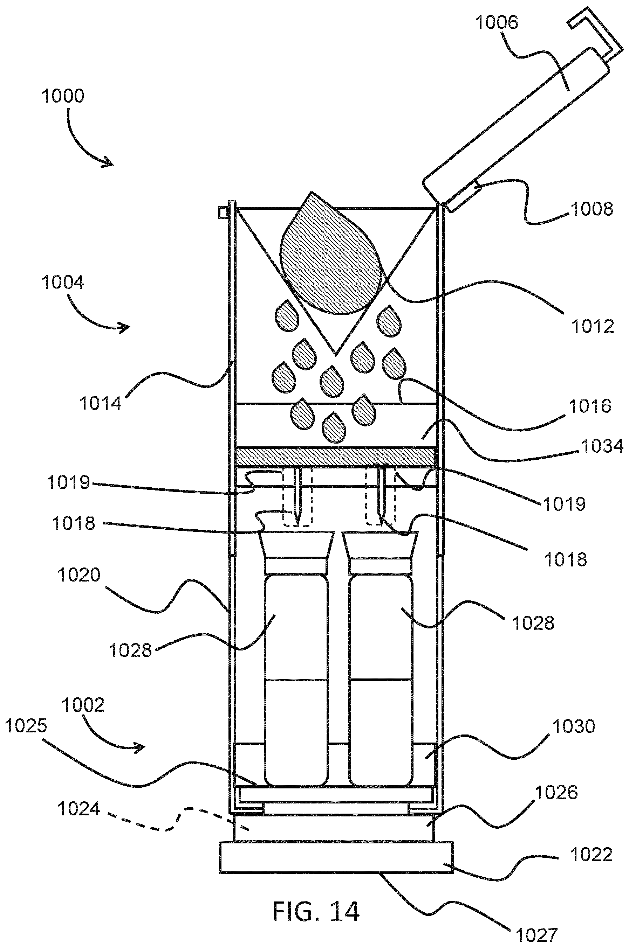

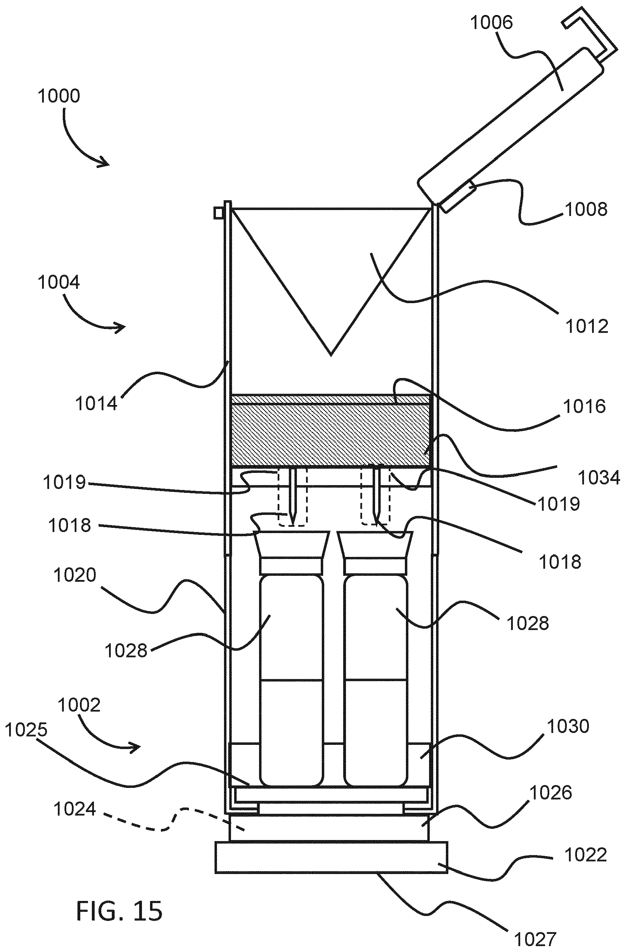

FIGS. 11-21 illustrate an exemplary method of using the oral fluid collection device **1000**. As illustrated in FIG. 11, when a donor is ready to use the oral fluid collection device **1000**, the donor will unsecure the protrusion and snap mechanism **1010** and rotate the cap **1006** about the hinge **1008** to open the oral fluid collection device **1000**. As illustrated in FIG. 12, the donor deposits oral fluid into the opening in the oral fluid collection device **1000**. As the oral fluid is deposited into the oral fluid collection device **1000**, it engages the filter funnel **1012** (as illustrated in FIG. 13), where the filter funnel **1012** filters out unwanted materials from the oral fluid. The oral fluid then progresses through filter funnel **1012** downward into a collection area **1034** (as illustrated in FIG. 14). As all the oral fluid filters through the filter funnel **1012** and into the collection area **1034**, the collected fluid may surpass the fill line **1016** on the collection cup **1004** (as illustrated in FIG. 15). If the oral fluid in the collection area **1034** does not surpass the fill line **1016** of the collection cup **1004** with the first oral fluid deposit from the donor, the donor can again deposit oral fluid into the oral fluid collection device **1000** until the oral fluid surpasses the fill line **1016**.

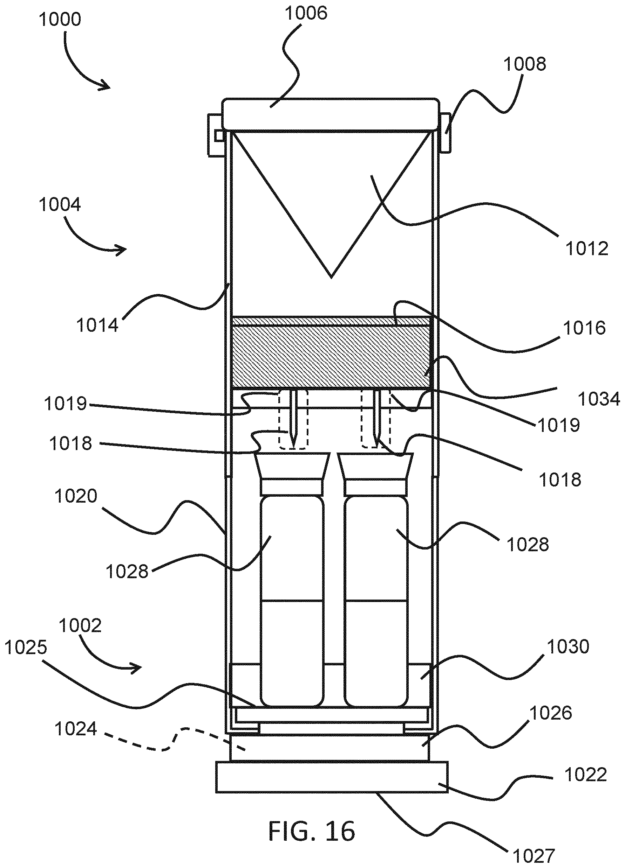

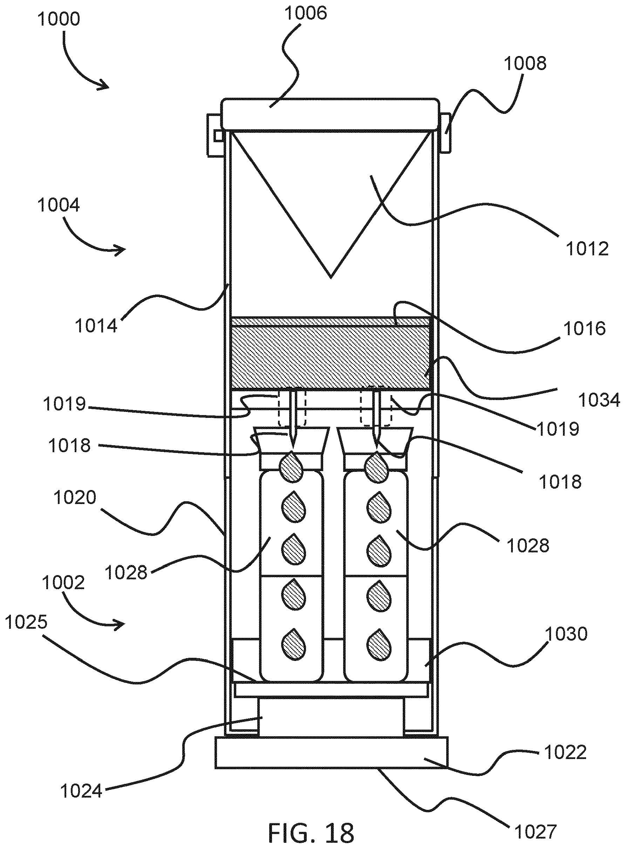

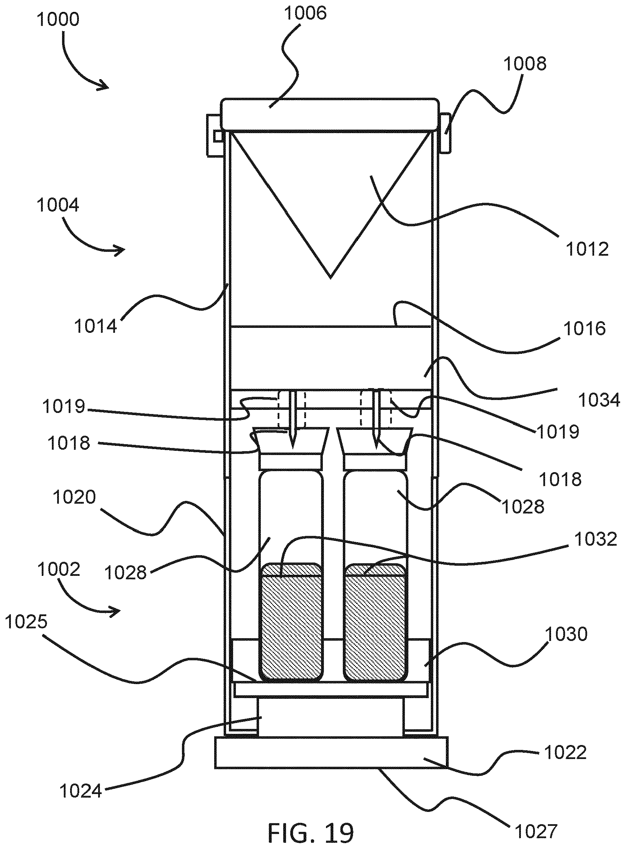

Once the fill line **1016** on the collection cup **1004** is surpassed, the cap **1006** is closed and latched (as illustrated in FIG. 16) and the removable tab **1026** is removed from the recessed section **1024** of the pedestal **1022** (as illustrated in FIG. 17). The oral fluid collection device **1000** is now in condition to fill the vacuum tubes **1028** with oral fluid in the collection area **1034**. As illustrated in FIG. 18, with the removable tab **1016** removed, the collection cup **1004** and thin walled body **1020** of the base **1002** can move downward relative to the vacuum tubes **1028**. Such movement moves the hollow needles **1018** downward and engages the hollow needles **1018** with the vacuum tubes **1028**; thus, facilitating the flow of oral fluid from the collection area **1034** into the vacuum tubes **1028**. As all the oral fluid flows from the collection area **1034** into the vacuum tubes **1028**, the oral fluid will surpass the vacuum tube fill lines **1032** (as illustrated in FIG. 19).

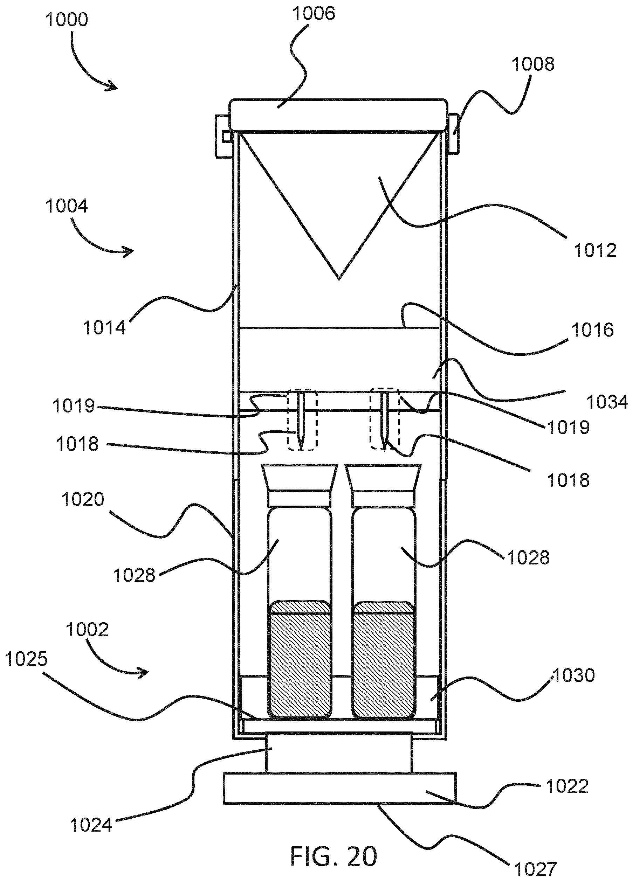

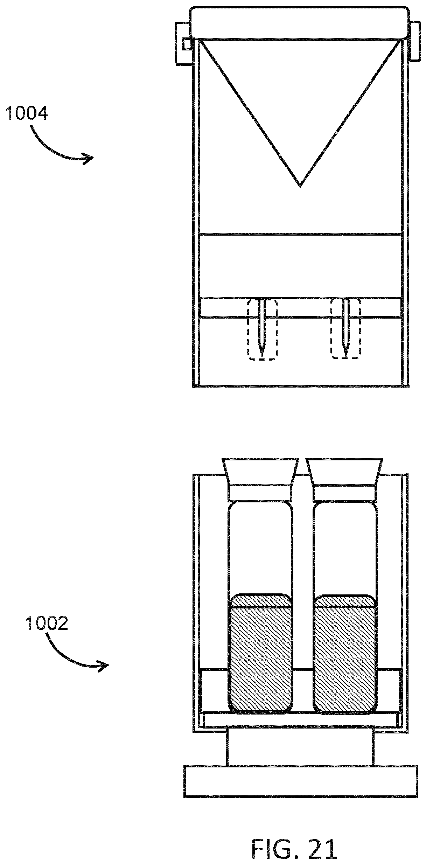

One method of moving the collection cup **1004** and thin walled body **1020** of the base **1002** downward relative to the vacuum tubes **1028** is to place the oral fluid collection device **1000** on a flat surface, such as a table top, with the lower surface **1027** of the pedestal **1022** in contact with the flat surface, and apply downward pressure on the top of the oral fluid collection device **1000** until the hollow needles **1018** engage with the vacuum tubes **1028** and oral fluid flows into the vacuum tubes **1028**. Once the vacuum tubes **1028** are filled, the oral fluid collection device **1000** can be returned to its original position (as illustrated in FIG. 20), any seal or mechanism joining the collection cup **1004** to the base **1002** is removed and the collection cup **1004** can be separated from the base **1002** (as illustrated in FIG. 21). The vacuum tubes **1028** can now be removed from the base **1002** and secured and packaged for shipping or transport to a laboratory for testing. Once the vacuum tubes are removed and secured, both the base **1002** and the collection cup **1004** can be discarded.

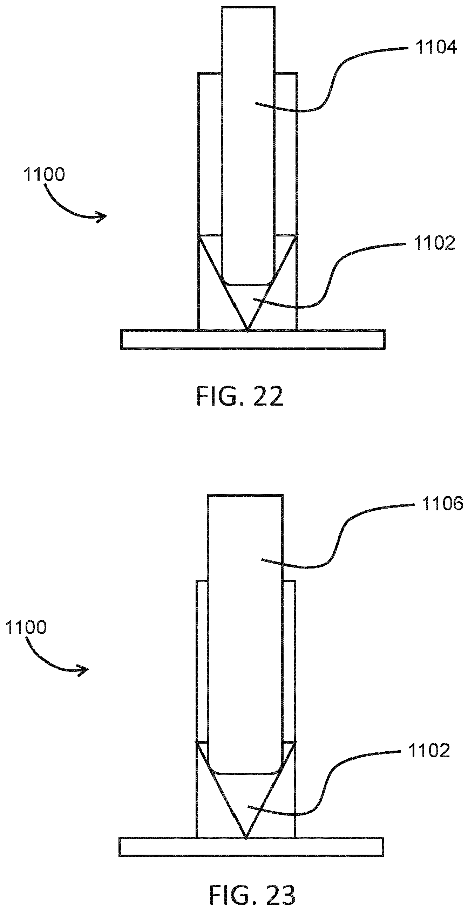

In other embodiments of oral fluid collection devices, a base of such a device can include features that provide for accommodating vacuum tubes of different sizes. Additionally, components and features can be incorporated into an oral fluid collection device to facilitate the engagement and disengagement of vacuum tubes and hollow needles and to protect users from the hollow needles. Such components and features include the use of a force generated by the compression of a resilient material to manage the engagement of the hollow needle with the vacuum tube and the use of a force generated by the release of a compressed material to manage the disengagement of the hollow needle from the vacuum tube. FIGS. 22-31 illustrate exemplary components that add such features and functionality, which can be used with any of the embodiments disclosed herein.

In one example of a base **1100**, schematically illustrated in FIGS. 22 and 23, the base **1100** can include a feature to accommodate different sized vacuum tubes. The exemplary base **1100** includes a cone shaped feature **1102** that can form a friction fit with a vacuum tube inserted into the base **1100**. As illustrated in FIG. 22, a vacuum tube **1104** with a relatively small diameter is accommodated by the cone-shaped feature **1102** in the base **1100**. As illustrated in FIG. 23, a vacuum tube **1106** with a relatively large diameter is accommodated by the cone shaped feature **1102** in the base **1100**. In addition to a cone shaped feature, the base can include a funnel shaped feature, a wedge shaped feature or any other shape that facilitates the insertion of a vacuum tube, including vacuum tubes of varying sizes.

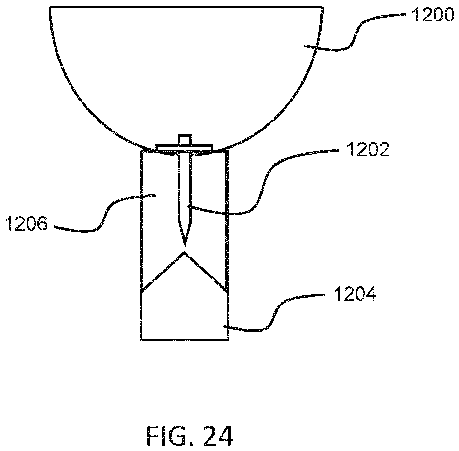

FIG. 24 schematically illustrates an exemplary collection cup **1200** with an integrated hollow needle **1202** and a tube guide **1204** extending downward from the collection cup **1200** and enclosing the hollow needle **1202**. Positioned within the tube guide **1204**, and surrounding the hollow needle **1202**, is a compressible and resilient sleeve **1206**. The sleeve **1206** consists of a material that can be deformed by an applied force, but will return to its original shape upon the removal of the force. Once such material is polypropylene; however, it will be understood that many materials can be used for the sleeve **1206**. As illustrated in FIG. 24, the sleeve **1206** occupies the available volume of the tube guide **1204** and extends beyond the hollow needle **1202**; thus, protecting both the hollow needle **1202** and any user or donor that handles the oral fluid collection device. Similar to the flexible, compressible, penetrable sleeves **116**, **1019** described above, the compressible and resilient sleeve **1206** illustrated in FIGS. 24-31 can prevent unwanted drainage through the hollow needle **1202**.

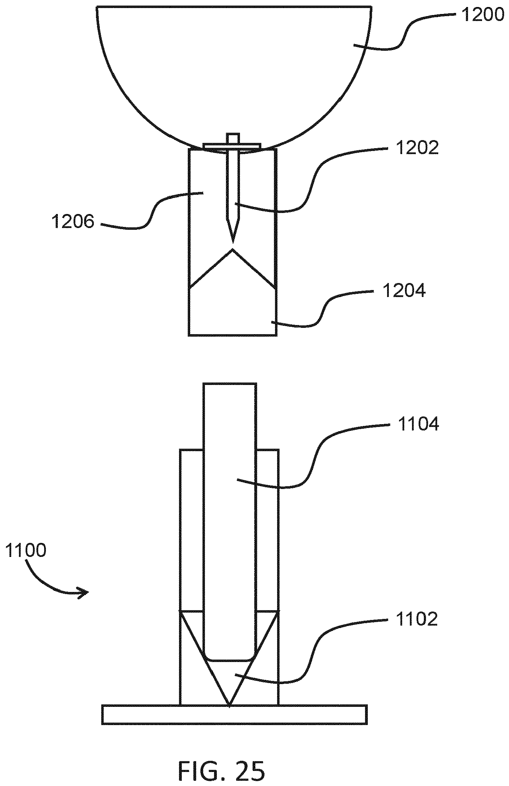

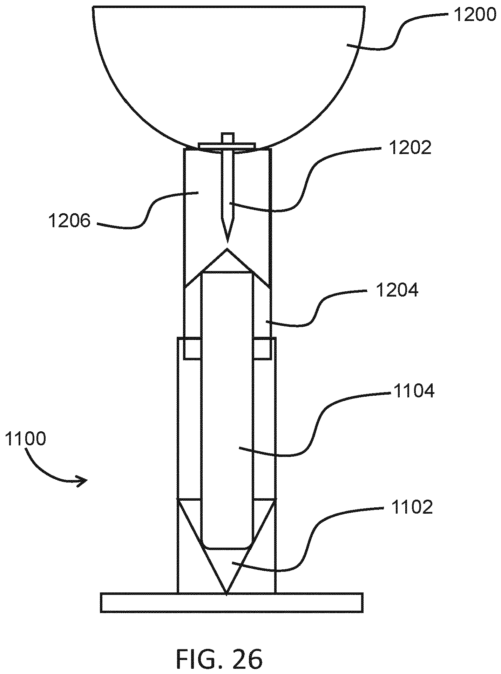

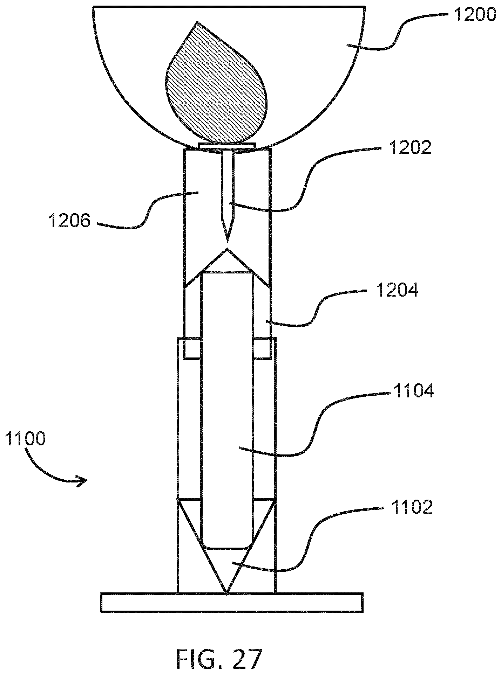

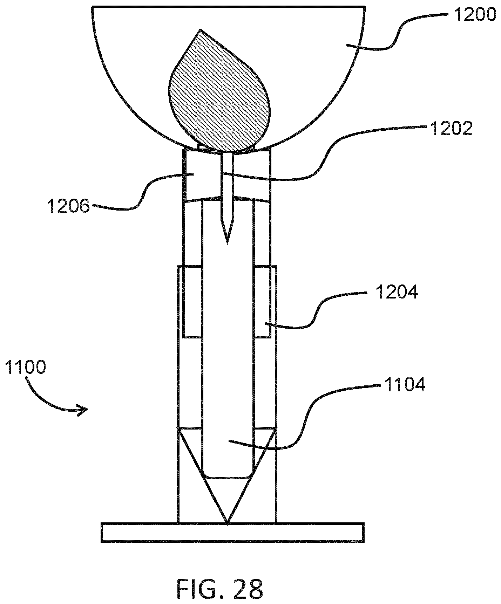

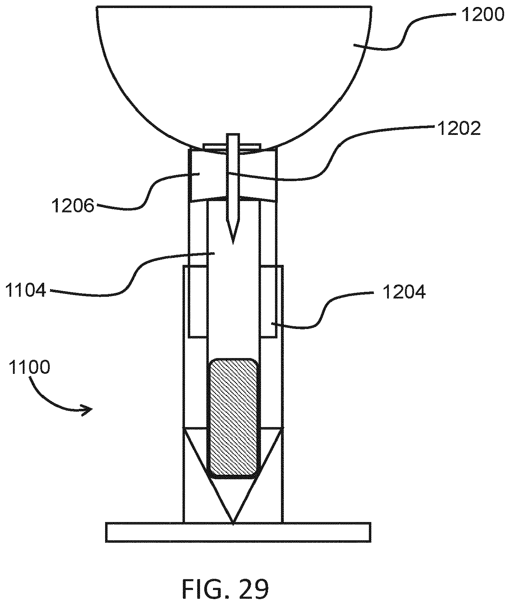

FIGS. 25-31 schematically illustrate the combination of the base **1100** of FIGS. 22 and 23, and the collection cup **1200** and related components of FIG. 24. When the base **1100** and collection cup **1200** (and related components) are to be used together, a user positions the collection cup **1200** over the base **1100**, with a vacuum tube **1104** positioned in the base **1100**. As illustrated in FIG. 26, the collection cup **1200** is lowered toward the base **1100**, and the tube guide **1204** is slid over the vacuum tube **1104** and into the base **1100**. The collection cup **1200** is lowered until the top of the vacuum tube **1104** is positioned near the bottom of the sleeve **1206**. Once in such a position, as illustrated in FIG. 27, the donor can deposit a specimen in the collection cup **1200**. Once a sufficient volume of oral fluid is deposited in the collection cup **1200**, the user or donor can further move the collection cup **1200** downward, applying enough force to overcome the resilient nature of the sleeve **1206** (as illustrated in FIG. 28). The force generated by the compression of the sleeve **1206** provides for smooth and controlled downward movement of the collection cup **1200**. As the sleeve **1206** compresses, the hollow needle **1202** engages the vacuum tube **1104** (i.e., pierces the membrane sealing the vacuum tube **1104**) and the vacuum within the vacuum tube **1104** draws the specimen into the vacuum tube **1104** through the hollow needle **1202** (as illustrated in FIG. 29).

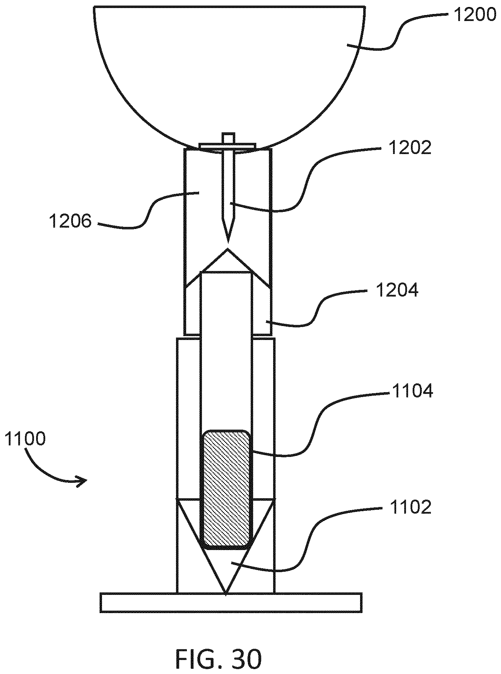

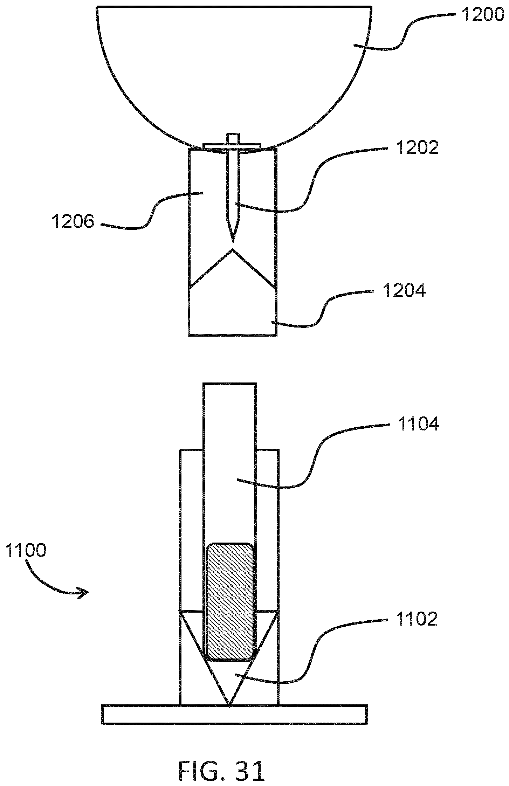

Once the user or donor releases the downward pressure on the collection cup **1200**, the resilient nature of the sleeve **1206** returns to its original shape and applies a force on the vacuum tube **1104**. As illustrated in FIG. 30, such a force applied by the sleeve **1206** moves the collection cup **1200** upward, and the hollow needle **1202** is moved upward and withdrawn from the membrane sealing the vacuum tube **1104**. Once the hollow needle **1202** and vacuum tube **1104** are disengaged, the collection cup **1200** can then be removed from the base **1100** (as illustrated in FIG. 31), and the user or donor can remove the vacuum tubes **1104** from the base **1100** and secure and package the vacuum tube **1104** for shipping or transport to a laboratory for testing. The sleeve **1206** once again covers the hollow needle **1202** to protect the user or donor when handling the oral fluid collection device. Once the vacuum tube **1104** is removed and secured, both the base **1100** and the collection cup **1200** can be discarded.

Embodiments may be defined in accordance with the following clauses:
1. An assembly for collecting oral fluids, saliva, or sputum, comprising:
   a collection cup;
   at least one hollow needle positioned at least partially in the collection cup;
   a base engaged with the collection cup, the base comprising:
      a thin walled body;
      a pedestal forming the bottom of the base; and
      a recessed section formed in the pedestal;
   at least one vacuum tube positioned in the base; and
   a removeable tab positioned in the recessed section.
2. The assembly of clause 1, wherein when the removeable tab is positioned in the recessed section of the pedestal, the positional relationship between the at least one hollow needle and the at least one vacuum tube is static.
3. The assembly of clause 1 or 2, wherein when the removeable tab is removed from the recessed section of the pedestal, the at least one hollow needle and the at least one vacuum tube can move relative to each other.
4. The assembly of any preceding clause, wherein the assembly further comprises a vacuum tube holder positioned within the base and arranged to reversibly engage the one or more vacuum tubes.
5. The assembly of any preceding clause, further comprising at least one penetrable sleeves, each covering one of the at least one hollow needle.
6. The assembly of any preceding clause, further comprising a filter arranged to be positioned in an interior of the collection cup.
7. The assembly of any preceding clause, wherein:
   the assembly further comprises a cap;
   a hinge to rotationally secure the cap to the collection cup; and
   a protrusion and snap mechanism for closing an opening of the collection cup.
8. The assembly of any preceding clause, wherein as the at least one hollow needle and the at least one vacuum tube move closer to each other, the at least one hollow needle engages with the at least one vacuum tube.
9. The assembly of any preceding clause, wherein:
   the at least one hollow needle comprises a first hollow needle and a second hollow needle;
   the at least one vacuum tubes comprise a first vacuum tube and a second vacuum tube; and
   the one or more penetrable seals comprises a first penetrable seal and a second penetrable seal.

The foregoing description illustrates and describes the disclosure. Additionally, the disclosure shows and describes only the preferred embodiments but, it is to be understood that the preferred embodiments are capable of being formed in various other combinations, modifications, and environments and are capable of changes or modifications within the scope of the invention concepts as expressed herein, commensurate with the above teachings and/or the skill or knowledge of the relevant art. The embodiments described herein above are further intended to explain the best modes known by applicant and to enable others skilled in the art to utilize the disclosure in such, or other, embodiments and with the various modifications required by the particular applications or uses thereof. Accordingly, the description is not intended to limit the invention to the form disclosed herein. Also, it is intended that the appended claims be construed to include alternative embodiments. It will be further understood that various changes in the details, materials, and arrangements of the parts which have been described and illustrated above in order to explain the nature of this invention may be made by those skilled in the art without departing from the principle and scope of the invention as recited in the following claims.

## Claims

1. An assembly for collecting oral fluids, saliva, or sputum, comprising:
a collection cup;
at least one hollow needle positioned at least partially in the collection cup;
a base engaged with the collection cup, the base comprising:
a thin walled body; and
a pedestal forming the bottom of the base positioned partially within the thin walled body and partially outside the thin walled body and arranged to move linearly relative to the thin walled body, the pedestal comprising:
an upper surface;
a lower surface; and
a recessed section located between the upper surface and lower surface;
at least one vacuum tube positioned at least partially within the thin walled body and in contact with the upper surface of the pedestal; and
a removeable tab optionally positioned in the recessed section of the pedestal.

2. The assembly of claim 1, wherein when the removeable tab is positioned in the recessed section of the pedestal, the positional relationship between the at least one hollow needle and the at least one vacuum tube is static, and when the removeable tab is removed from the recessed section of the pedestal, the at least one hollow needle and the at least one vacuum tube can move relative to each other.

3. The assembly of claim 1 or 2, wherein the assembly further comprises a vacuum tube holder positioned within the base and arranged to reversibly engage the one or more vacuum tubes.

4. The assembly of any preceding claim, further comprising one or more penetrable sleeves, each covering one of the at least one hollow needle.

5. The assembly of any preceding claim, further comprising:
a filter arranged to be positioned in an interior of the collection cup
a cap;
a hinge to rotationally secure the cap to the collection cup; and
a protrusion and snap mechanism for closing an opening of the collection cup.

6. The assembly of any preceding claim, wherein as the at least one hollow needle and the at least one vacuum tube move closer to each other, the at least one hollow needle engages with the at least one vacuum tube.

7. The assembly of any preceding claim, wherein:
the at least one hollow needle comprises a first hollow needle and a second hollow needle; and
the at least one vacuum tubes comprise a first vacuum tube and a second vacuum tube.

8. The assembly of claim 7, further comprising:
a first penetrable seal positioned to seal a top end of the first vacuum tube; and
a second penetrable seal positioned to seal a top end of the second vacuum tube.

9. The assembly of claim 7 or 8, wherein when the removeable tab is positioned in the recessed section of the pedestal, the positional relationship between the first hollow needle and the first vacuum tube is static and the positional relationship between the second hollow needle and the second vacuum tube is static, and wherein when the removeable tab is removed from the recessed section of the pedestal, the first hollow needle and the first vacuum tube can move relative to each other and the second hollow needle and the second vacuum tube can move relative to each other.

10. The assembly of claim 8 or any claim dependent thereon, wherein:
as the first hollow needle and the first vacuum tube move closer to each other, the first hollow needle penetrates the first penetrable seal; and
as the second hollow needle and the second vacuum tube move closer to each other, the second hollow needle penetrates the second penetrable seal.

11. The assembly of any of claims 7 to 10, wherein the assembly further comprises a vacuum tube holder positioned within the base and arranged to reversibly engage the first vacuum tube and the second vacuum tube.

12. The assembly of any of claims 7 to 11, further comprising:
a filter arranged to be positioned in an interior of the collection cup
a cap;
a hinge to rotationally secure the cap to the collection cup; and
a protrusion and snap mechanism for closing an opening of the collection cup.

13. The assembly of any of claims 7 to 12, further comprising a first penetrable sleeve covering the first hollow needle and a second penetrable sleeve covering the second hollow needle.

14. The assembly of any of claims 7 to 13 further comprising a resilient sleeve positioned in a bottom end of the collection cup, wherein when the resilient sleeve is in an uncompressed state, the first hollow needle and a second hollow needle are positioned within the resilient material and when the collection cup and the base are moved toward each other, the resilient sleeve compresses and applies a force resisting the collection cup and base moving toward each other.

15. The assembly of any of claims 1 to 6 further comprising a resilient sleeve positioned in a bottom end of the collection cup, wherein when the resilient sleeve is in an uncompressed state, the first hollow needle and a second hollow needle are positioned within the resilient sleeve and when the collection cup and the base are moved toward each other, the resilient sleeve compresses and applies a force resisting the collection cup and the base moving toward each other.
